# EUROPÄISCHE PATENTSCHRIFT

(11) **EP 1 759 730 B1**
(45) Veröffentlichungstag und Bekanntmachung des Hinweises auf die Patenterteilung: **09.03.2016**
(21) Anmeldenummer: 06026254.0
(22) Anmeldetag: 22.07.2002
(51) Int. Cl.: A61M 5/34

(54) **Befestigungsvorrichtung für Injektionsnadeln**
Attachment device for injection needles
Dispositif d'attache pour aiguilles d'injection

(30) Priorität: 21.08.2001 CH 15432001
(43) Veröffentlichungstag der Anmeldung: 07.03.2007
(62) Teilanmeldung aus: 02745035.2
(73) Patentinhaber: TecPharma Licensing AG, 3401 Burgdorf (CH)
(72) Erfinder: Covino, Gian-Carlo, 4103 Bottmingen (CH); Fiechter, Patrick, 4950 Huttwil (CH); Kirchhofer, Fritz, 3454 Sumiswald (DE); Lehrmann, Hans-Ulrich, 2564 Bellmund (CH); Scheidegger, Daniel, 3011 Bern (CH); Wenger, Stefan, 3098 Schliern bei Koeniz (CH)
(74) Vertreter: Schwabe - Sandmair - Marx

(56) Entgegenhaltungen:
- EP-A1- 1 420 840
- GB-A- 737 676
- US-A- 2 828 743
- US-A- 2 894 509
- US-A- 4 281 653
- US-A- 5 611 786
- US-A- 5 782 803

## Beschreibung

Die Erfindung bezieht sich auf Befestigungsvorrichtungen für Injektionsnadeln gemäss Oberbegriff des Patentanspruchs 1.

In der ambulanten Medizin werden zur subkutanen Verabreichung von flüssigen Medikamenten zunehmend an Stelle von Einwegspritzen wiederverwendbare Injektionsgeräte eingesetzt, zum Beispiel ein Injektionspen, welcher das Medikament in der gewünschten Menge mittels einer Dosiereinrichtung aus einer vorgefüllten Ampulle abgibt. Pro Injektion wird in der Regel jeweils auch eine neue Injektionsnadel, in der Folge einfach Nadel genannt, auf den Injektionspen aufgesetzt und nach deren Verwendung wieder entfernt. Die Nadel ist eine Hohlnadel zur Durchleitung von Arzneimittel von einem Vorratsbehälter in den Körper. Trotz gewisser Normen für Injektionsgeräte, bevorzugen die Hersteller Injektionsgeräte zu entwickeln, welche spezifisch nur für ihre Medikamente geeignet sind. Somit hat praktisch jedes dieser Injektionsgeräte eine spezifische Befestigungsvorrichtung für die Nadel, welche dadurch nicht oder nur unsicher auf ein anderes Modell passt. Eine sichere Befestigung der Nadel auf einem Injektionspen ist dann, wenn diese weder rutscht noch aus dem Lot kippt. Eine unsicher befestigte Nadel erhöht das Risiko von Verletzungen oder eines Nadeibruchs. Falls ein Arzt oder Patient mehrere Medikamente bzw. Injektionsgeräte für eine Therapie benötigt, ist er zudem auf eine entsprechende Vielfalt an Nadeln angewiesen, was deren Beschaffung und Lagerhaltung erschwert und bei kleineren Mengen auch teurer macht
Folglich war man auf der Suche nach einer Befestigungsvorrichtung für Injektionsnadeln, welche die Nadel auf unterschiedlichen Modellen von Injektionsgeräten sicher und lösbar befestigt, insbesondere auf den handelsüblichen Injektionspens mit einem Gewinde oder mit Gewindesegmenten zur lösbaren Befestigung der Nadel.
Befestigungsvorrichtungen für Injektionsnadeln zum Aufstecken oder Aufschrauben der Nadel auf das Gewinde oder Gewindesegment sind beispielsweise aus der Schrift WO 95/01812 und GB 737,676 bekannt. Eine typische Befestigungsvorrichtung hat die Form einer Kappe mit einem offenen und einem geschlossenen, mittig senkrecht die Nadel haftenden Ende. An der Innenseite der Mantelflächen dieser Kappe befindet sich mindestens eine Erhebung oder Vertiefung, zum Beispiel ein Nocken, ein Gewinde oder ein Gewindesegment, die in den Gewindegang des komplementären Gewindes oder Gewindesegments des Injektionspens einschnappt oder durch Drehen eingeschraubt wird. Die Befestigungsvorrichtungen aus den oben genannten Patentschriften haben typischerweise, sich zum Gewinde hin verjüngende Nocken mit einer, dem Gewinde angepassten Steigung, so dass, beim Aufstecken auf ein Gewinde mit unterschiedlicher Steigung, die Kappe unerwünscht aus dem Lot kippt, falls die Nocken in den Gewindegang eingreifen.
Um das Kippen zu verhindern, braucht es mindestens drei Kontaktpunkte zwischen der Innenseite der Kappe bzw. deren Nocken und dem Gewinde des Injektionspens, welche nicht in der gleichen Ebene sind und zudem die Kappe im Lot halten. Das heisst, dass diese Kontaktpunkte nach dem Aufstecken sich nicht mehr verschieben dürfen, ansonsten die Nadel entlang der Richtung der Nadel rutscht. Obschon nicht für Injektionspens vorgesehen, beschreiben die Patentschriften US 2,894,509 und US 2,828,743 Befestigungsvorrichtungen mit Nocken bzw. Nuten, welche unabhängig von einer Gewindesteigung, nicht in der gleichen Ebene sind, und zwei feste Positionen und eine definierte Verschiebung einer Nadel ermöglichen. Die Lösung aus der Schrift US 2,828,743 ist jedoch mit den praktisch den ganzen Umfang umschliessenden Nuten für das lösbare Aufstecken oder Aufschrauben auf ein Gewinde nicht geeignet. Die Lösung aus Figur 2 und 3 der Schrift US 2,894,509 ist nur dann für ein Gewinde oder für Gewindesegmente geeignet, wenn die Nocken gleichmässig und rutschfest in die Gewindegänge eingreifen, was bei den unterschiedlichen Dimensionen der Gewinde selten der Fall Ist. In besonderen Fällen kann auch diese Kappe aus dem Lot kippen, wobei das obere oder das untere Nockenpaar eine Kippachse beschreiben. Folglich wären mehr Nocken notwendig, um dieser Kippsymmetrie entgegen zu wirken. Mehr Nocken ergibt ferner den Vorteil, dass durch mehr Kontaktpunkte, die Klemmkraft in diesen Punkten kleiner sein darf, bei gleichbleibender Haftung der Kappe bzw. der Nadel auf dem Injektionspen. Die Klemmkraft entspricht einer Federkraft, welche vorwiegend durch die Geometrie der Nocken, der Anordnung auf der Mantelfläche der Kappe und der Elastizität der Werkstoffe bestimmt wird. Eine kleinere Klemmkraft erlaubt daher bei gleichbleibender Federkraft einen grosseren Federweg der Nocken oder der Mantelfläche der Kappe. Folglich würde eine soiche Kappe auf entsprechend grössere oder kleinere Gewindedurchmesser passen, vorausgesetzt die Klemmkraft wirkt stets gleich, vorzugsweise senkrecht auf das Gewinde des Injektionspens. Keine der bekannten Befestigungsvorrichtungen für Injektionsnadeln zeigt eine Lösung, bei der die Federkräfte in den Kontaktpunkten bei erhöhtem Fadenweg stets gleich, insbesondere senkrecht, auf das Gewinde wirken. Dies wäre der Fall, wenn die Nocken in der Mitte von federnden Elementen angebracht wären, welche mit je einem Ende, analog eines Dreipunkt-Biegeelements mit der formfesten Mantelfläche verbunden wären. Eine Verdoppelung der Kontaktpunkte, um einen grösseren Federweg der Nocken für ebenso grössere Unterschiede In den Durchmessem der Gewinde zu ermöglichen, erfordert mindestens doppelt so viel Platz auf der Innenseite der Mantelfläche von der Kappe. Dies ist bei den bekannten Lösungen mit bis zu maximal fünf Nocken konstruktiv sehr aufwendig oder gar nicht lösbar, bedenkt man, dass die Nocken auf unterschiedlichen Ebenen angeordnet sein müssen, falls die Kappe nicht aus dem Lot kippen soll. Desweiteren ist aus der Patentschrift US-A-5,611,786 eine Befestigungsvorrichtung für Injektionsnadeln zum Aufstecken auf ein Gewinde eines Injektionsgerätes gemäß dem Oberbegriff des Anspruchs 1 bekannt.

Die Erfindung hat die Aufgabe, eine Befestigungsvorrichtung für Injektionsnadeln mit mindestens 6 möglichen Kontaktpunkten auf mindestens zwei Ebenen zu schaffen, wobei die Federkräfte in den Kontaktpunkten vorzugsweise stets senkrecht auf das Gewinde des Injektionsgerätes wirken, damit die Nadel sicher auf mehrere Modelle von Injektionspens passt.

Die erfindungsgemässe Befestigungsvorrichtung für Injektionsnadeln hat mindestens drei Nocken, deren sich zum Gewinde hin verjüngende Spitzen je mindestens eine parallel zum Mantelumfang angeordnete Kerbe aufweisen, womit jeder Nocken mindestens zwei ebenfalls senkrecht ins Gewinde eingreifende Spitzen hat. Diese Lösung ergibt mindestens drei, nicht in der gleichen Ebene liegende Klemmpunkte zwischen der Kappe und dem Gewinde, welche für einen festen Sitz der Kappe auf dem Gewinde erforderlich sind. Zudem reduzieren die zusätzlichen Klemmpunkte die benötigte Klemmkraft oder ermöglichen eine entsprechend höhere Elastizität der Nocken bzw. der Mantelfläche der Kappe, damit die Kappe bzw. die Nadel auf entsprechend grössere und kleinere Gewindedurchmesser passt.
Bei einer bevorzugten Ausführung der erfindungsgemässen Befestigungsvorrichtung für Injektionsnadeln ist die Distanz zwischen den Spitzen eines jeden gekerbten Nockens mindestens die Höhe des grössten Gewindegangs, wornit die Kappe ohne Rutschen oder Verkanten auf die entsprechenden Injektionspens mit unterschiedlicher Steigung und Form von Gewindegängen passt.

Vorteilhaft für die erfindungsgemässe Befestigungsvorrichtung ist zudem, wenn die Mantelfläche der Kappe in der Höhe nicht elastisch verformbar ist, sondern daran Federelemente befestigt sind, welche die gekerbten Nocken halten. Vorzugsweise wirken diese Federelemente in der Art von Biegebalken, auf deren Mittelpunkte je ein gekerbter Nocken befestigt ist, womit dessen Spitzen stets optimal, das heisst senkrecht in die Gewindegänge eingreifen.

Trotz der verbesserten Haftung der Injektionsnadel auf einem handelsüblichen Injektionsgerät, entspricht eine nach der Erfindung konstruierte Befestigungsvorrichtung dem üblichen Aufwand für die Konstruktion und Herstellung für Kappen, welche eine Injektionsnadel haften. Folglich lässt sich eine erfindungsgemässe Befestigungsvorrichtung für injektionsnadeln ebenso kostengünstig und in grossen Serien als einstückiges Spritzgussteil aus Kunststoff herstellen. Durch seine gute Formbeständigkeit ist PCTG (Polycyclohexylen-dimethylen-therephtalat) für diese Anwendung ein besonders geeignetes Material.

Eine besonders interessante Anwendung der erfindungsgemässen Befestigungsvorrichtung für Injektionsnadeln ist die ambulante Insuilntherapie für Diabetiker, wobei die meisten Injektionspens je nach Therapie oft in diversen Kombinationen verwendet werden. Da diese Injektionspens als Medikamentenbehälter Ampullen haben, welche mit einem Septum bzw. einer Gummimembran verschlossen sind, muss das eine Ende der Nadel in die Kappe hineinragen, so dass diese beim Aufstecken der Kappe auf das Gewinde das Septum durchsticht. Bedingt durch ähnliche, das Septum haltende Verschlüsse der Ampullen, haben diese Injektionspens einen Aussendurchmesser des Gewindes oder Gewindesegments im Bereich von 9 bis 10mm. Gemäss der Erfindung passt eine Kappe mit einem entsprechend dimensionierten Innendurchmesser und mit mindestens fünf, regelmässig Ober den Umfang verteilten Federelementen und gekerbten Nocken sicher auf praktisch alle handelsüblichen Injektionspens für Insulin.

Vorzugsweise handelt es sich bei der Nadel um eine 30 Gauge oder 31 Gauge Nadel. Besonders bevorzugt um eine Nadel kleiner als 31 Gauge.

Bevorzugte Ausführungsbeispiele der erfindungsgemässen Befestigungsvorrichtung sind nachstehend mit Hilfe von Figuren beschrieben, wobei die Figuren folgendes darstellen:
- Fig. 1: einen ersten schematisch dargestellten Vergleich zwischen einer bestehenden und einer erfindungsgemässen Befestigungsvorrichtung für Injektionsnadeln;
- Fig. 2: einen zweiten schematisch dargestellten Vergleich zwischen einer bestehenden und einer erfindungsgemässen Befestigungsvorrichtung für Injektionsnadeln;
- Fig. 3: einen Vertikalschnitt durch eine erfindungsgemässe Befestigungsvorrichtung für Injektionsnadeln und
- Fig. 4: eine Teilansicht einer schematischen Schnittzeichnung einer auf einem Injektionspen aufgesteckten Injektionsnadel mit einer erfindungsgemässen Befestigungsvorrichtung.

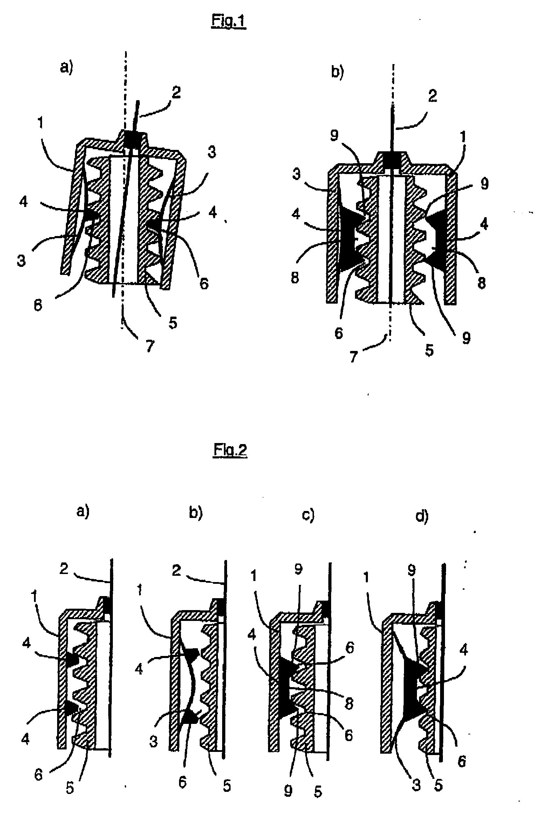

Die Figur 1 zeigt schematisch einen Vergleich zwischen einer bestehenden, wie in der Teilzeichnung a) dargestellt ist, und einer erfindungsgemässen Befestigungsvorrichtung bzw. Kappe 1 für eine Nadel 2, wie dies in der Teilzeichnung b) dargestellt ist. Die Kappen 1 haben je ein unteres, offenes Ende und ein oberes, geschlossenes Ende, welches die Nadel 2 mittig und senkrecht hält. An der inneren Mantelfläche der Kappen 1 sind Federelemente 3 in der Art von Biegebalken angeordnet, in deren Mitte sich je ein Nocken 4 befindet. Wird nun die Kappe 1 über ein Gewinde 5 gestülpt, so greifen in der bestehenden Lösung a) die Nocken 4 in den Gewindegang 6 des Gewindes 5. Ist die Positionierung der Nocken 4 nicht der Steigung des Gewindegangs 6 angepasst, so kippt die Kappe 1 nachteilig aus dem Lot bzw. die Nadel 2 ist nicht mehr parallel zur Rotationsachse 7 des Gewindes 5. In der efindungsgemässen Lösung b) sind die Nocken 4 analog auf Federelementen 3 angebracht, haben aber, im Gegensatz zu den bestehenden Nocken 4, kein einfaches, sich zum Gewinde 5 hin verjüngendes Ende. Dieses hat durch eine, horizontal zur Mantelfläche der Kappe 1 verlaufenden Kerbe 8 zwei Spitzen 9, welche je senkrecht In den Gewindegang 6 einwirken. Der Abstand der Spitzen 9 eines Nockens 4 ist mindestens als die Höhe des Gewindegangs 6, was mindestens drei, nicht in der gleichen Ebene liegende Kontaktpunkte zwischen der erfindungsgemässen Befestigungsvorrichtung bzw. Kappe 1 und dem Gewinde 5 ergibt. Die Nadel 2 wird dadurch sicher, ohne aus dem Lot bzw. der Rotationsachse 7 des Gewindes 5 zu kippen gehalten.

In Figur 2 wird schematisch ein weiterer Vorteil der erfindungsgemässen Befestigungsvorrichtung bzw. Kappe 1 mit einem gekerbten Nocken 4, wie dies in der Teilzeichnung c) und d) dargestellt ist, gegenüber einer Kappe 1 mit zwei üblichen Nocken 4 gezeigt, wie dies In der Teilzeichnung a) und b) dargestellt ist. Das Federelement 3 in der unvorteilhaften Lösung a) und b), das in der Art eines Blegebalkens zwischen den beiden Enden der Kappe 4 angeordnet ist, hat zwei, mindestens die Höhe des Gewindegangs 6 beabstandete Nocken 4. Diese Nocken 4 wirken nur senkrecht auf ein Gewinde 5 bei entsprechendem Gewindedurchmesser, wie dies in der Teitzeichnung a) zu sehen ist. In der Teilzeichnung b) ist der Durchmesser des Gewindes 5 kleiner, wodurch sich das Federelement 3 mehr in Richtung des Gewindes 5 wölbt. Die sich darauf befindenden Nocken 4 kippen entsprechend der Wölbung des Federelements 3 aus der senkrechten Ausrichtung auf das Gewinde. Folglich wirkt die Klemmkraft nicht mehr optimal, das heisst, senkrecht auf das Gewinde 5, womit die Kappe 1 weniger sicher auf dem Gewinde 5 haftet. Die in der Teilzeichnung c) und d) gezeigte, erfindungsgemässe Lösung hat ein analoges, in der Art eines Biegebalken wirkendes Federelement 3, auf dessen Mitte ein Nocken 4 angeordnet ist. Gemäss der Erfindung hat dieser Nocken 4 eine Kerbe 8 und zwei zum Gewinde 5 gerichtete Spitzen 9, deren Abstand mindestens die Höhe des Gewindegangs 6 beträgt. Die Spitzen 9 bzw. die Federkraft wirkt vorteilhaft stets senkrecht und mit maximaler Federkraft auf das Gewinde 5 auch dann, wenn der Durchmesser des Gewindes 5 kleiner ist, wie dies in der Teilzeichnung d) gegenüber der Teilzeichnung c) gezeigt wird.

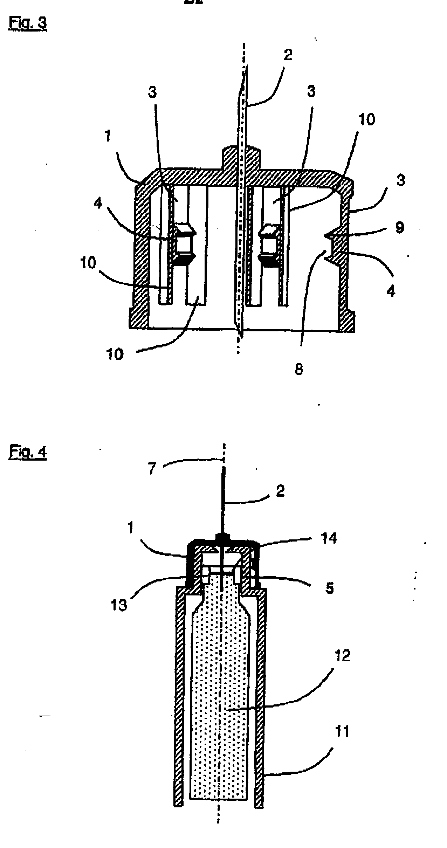

Die Figur 3 zeigt einen Horizontalschnitt mittig durch eine Befestigungsvorrichtung für Injektionsnadeln 2 gemäss der Erfindung, wobei die Kappe 1 unterschiedlich dicke Segmente längs der Manteffläche hat. Schlitze 10, die nicht ganz vom oberen zum unteren Ende der Kappe 1 reichen, trennen die dünneren Segmente beidseitig von den dickeren, wodurch sich die dünnen Segmente analog einem Blegebalken elastisch verformen können. Die so aus dem gleichen Material wie die Kappe 1 geformten Federelemente 3 weisen in der Mitte einen Nocken 4 auf, welcher eine steife, aus dem gleichen Material geformte Verdickung mit einer horizontalen Kerbe 8 und zwei Spitzen 9 ist.

Die Figur 4 ist eine Teilansicht einer schematischen Schnittzeichnung eines Injektionspens 11 mit Injektionsnadel 2, welche mit Hilfe einer erfindungsgemässen Befestigungsvorrichtung bzw. Kappe 1 sicher auf das Gewinde 5 aufgesteckt oder aufgeschraubt ist. Eine rotationssymmetrische Ampulle 12, zum Beispiel ein mit dem Medikament befüllte, zylindrischer Vorratsbehälter aus Glas oder Kunststoff, wird in dem dafür vorgesehen Teil des Injektionspens 11 eingesetzt. Die Ampulle 12 hat am einen Ende einen Verschluss 13, welcher üblicherweise in das Gewinde 5 des Injektionspens 11 hineinragt. Ein Teil des Verschlusses 13 ist ein Septum 14 bzw. eine Gummimembran, welche beim Aufstecken der Kappe 1 von der Nadel 2 durchstochen wird, falls das eine Ende der Nadel 2 entsprechend weit in das inner der Kappe 1 ragt. Anschliessend wird das Medikament mit einer Dosierelnrichtung, welche nicht gezeigt Ist, aus der Ampulle 12 in die Nadel 2 gefördert. Nach der Anwendung des Injektionspens 11 wird die Nadel 2 bzw. die Kappe 1 entfernt, wobei sich das Septum 14 wieder schliesst.

### Hinweisnummernverzeichnis

- 1: Kappe
- 2: Nadel
- 3: Federelement
- 4: Nocken
- 5: Gewinde
- 6: Gewindegang
- 7: Rotationsachse
- 8: Kerbe
- 9: Spitze
- 10: Schlitz
- 11: Injektionspens
- 12: Ampulle
- 13: Verschluss
- 14: Septum

## Patentansprüche

1. Befestigungsvorrichtung für Injektionsnadeln zum Aufstecken auf ein Gewinde (5) eines Injektionsgerätes, wobei die Befestigungsvorrichtung als Kappe (1) mit einem offenen und einem geschlossenen, mittig senkrecht eine Nadel (2) haltenden, Ende geformt ist, deren im Umfang leicht elastisch federnde Mantelfläche mindestens drei zum Gewinde (5) gerichtete Nocken (4) aufweist, welche beim Aufstecken der Kappe (1) auf das Gewinde (5) in die Gewindegänge (7) eingreifen, **dadurch gekennzeichnet, dass** die mindestens drei Nocken (4) mit dem Gewinde (5) mindestens sechs Kontaktpunkte auf mindestens zwei Ebenen bilden.

2. Befestigungsvorrichtung für Injektionsnadeln nach Anspruch 1, **dadurch gekennzeichnet, dass** jeder Nocken (4) mindestens zwei senkrecht ins Gewinde (5) eingreifende Spitzen (9) hat.

3. Befestigungsvorrichtung für Injektionsnadeln nach Anspruch 2, **dadurch gekennzeichnet, dass** jeder Nocken (4) mindestens eine parallel zum Mantelumfang angeordnete Kerbe (8) aufweist, wobei die Spitzen (9) der gekerbten Nocken (4) die mindestens sechs Kontaktpunkte auf mindestens zwei Ebenen bilden.

4. Befestigungsvorrichtung für Injektionsnadeln nach Anspruch 3, **dadurch gekennzeichnet, dass** die Distanz zwischen den Spitzen (9) eines jeden gekerbten Nockens (4) mindestens gleich der Höhe des Gewindeganges (7) ist.

5. Befestigungsvorrichtung für Injektionsnadeln nach Anspruch 3 oder 4, **dadurch gekennzeichnet, dass** die Mantelfläche der Kappe (1) in der Höhe nicht elastisch verformbar ist, und dass daran Federelemente (3) befestigt sind, welche die gekerbten Nocken (4) halten.

6. Befestigungsvorrichtung für Injektionsnadeln nach Anspruch 5, **dadurch gekennzeichnet, dass** die Federelemente (3) im Bereich des oberen und unteren Endes der Kappe (1) mit dieser verbunden sind, womit die Federelemente (3) Biegebalken sind, auf deren Mittelpunkte je ein gekerbter Nocken (4) befestigt ist, dessen Spitzen (9) stets senkrecht in die Gewindegänge (7) eingreifen.

7. Befestigungsvorrichtung für Injektionsnadeln nach Anspruch 5 oder 6, **dadurch gekennzeichnet, dass** die Federelemente (3) stoffgleiche Segmente der Mantelfläche der Kappe (1) sind, die am oberen und unteren Ende mit der Kappe (1) stofflich verbunden sind und eine elastische verformbare, entsprechende kleinere Wandstärke als die der festen Mantelfläche der Kappe (1) haben, und die mittig stoffgleiche, gekerbte und feste Nocken (4) aufweisen, deren Spitzen (9) senkrecht in die Gewindegänge (7) greifen.

8. Befestigungsvorrichtung für Injektionsnadeln nach einem der Ansprüche 5 bis 7, **dadurch gekennzeichnet, dass** die Kappe (1), deren Federelemente (3) und gekerbten Nocken (4) aus einem Teil gefertigt sind, vorzugsweise als Spritzgussteil.

9. Befestigungsvorrichtung für Injektionsnadeln nach Anspruch 8, **dadurch gekennzeichnet, dass** das Spritzgussmaterial ein thermoplastischer Kunststoff ist, vorzugsweise PCTG (Polycyclohexylen-dimethylen-therephtalat).

10. Befestigungsvorrichtung für Injektionsnadeln nach Anspruch 1 bis 9, **dadurch gekennzeichnet, dass** ein Ende der Nadel (2) in die Kappe (1) hineinragt, so dass diese beim Aufstecken der Kappe (1) auf das Gewinde (5) der Injektionseinheit in den mit Medikament befüllten Vorratsbehälter eindringt.

11. Befestigungsvorrichtung für Injektionsnadeln nach Anspruch 1 bis 10, **dadurch gekennzeichnet, dass** die Kappe (1) mindestens fünf Federelemente (3) mit gekerbten Nocken (4) regelmäßig über den Umfang verteilt aufweist und einen Innendurchmesser von mindestens 9 mm hat.

12. Befestigungsvorrichtung für Injektionsnadeln nach Anspruch 1 bis 11, **dadurch gekennzeichnet, dass** die Nadel (2) eine Hohlnadel kleiner als 30 Gauge ist.

## Claims

1. Attachment device for injection needles, for pushing onto a thread (5) of an injection apparatus, wherein the attachment device is formed as a cap (1) with one open and one closed end, centrally and vertically holding a needle (2), the circumference sleeve surface of which is slightly elastic and comprises at least three cams (4) facing in the direction of the thread (5), which engage the thread channels (7) when the cap (1) is pushed onto the thread (5), **characterised in that** the at least three cams (4) form at least six contact points on at least two levels with the thread (5).

2. Attachment device for injection needles according to claim 1, **characterised in that** each cam (4) has at least two tips (9) that vertically engage the thread (5).

3. Attachment device for injection needles according to claim 2, **characterised in that** each cam (4) comprises at least one groove (8) arranged parallel to the sleeve circumference, wherein the tips (9) of the grooved cams (4) form the at least six contact points on at least two levels.

4. Attachment device for injection needles according to claim 3, **characterised in that** the distance between the tips (9) of each grooved cam (4) is at least equal to the height of the thread channel (7).

5. Attachment device for injection needles according to claim 3 or 4, **characterised in that** the sleeve surface of the cap (1) is not elastically deformable in its height, and **in that** spring elements (3) are attached to the same, which hold the grooved cams (4).

6. Attachment device for injection needles according to claim 5, **characterised in that** the spring elements (3) are connected with the same in the areas of the upper and lower ends of the cap (1), which means that the spring elements (3) are bending beams, at the centre of each of which a grooved cam (4) is attached, the tips (9) of which always vertically engage the thread channels (7).

7. Attachment device for injection needles according to claim 5 or 6, **characterised in that** the material of the spring element (3) segments, which are materially connected at the upper and lower end with the cap (1) and have an elastically deformable, correspondingly smaller wall thickness than that of the fixed sleeve surface of the cap (1), and which comprise the grooved and fixed cams (4) of a centrally identical material, the tips (9) of which vertically engage the thread channels (7), is identical to that of the sleeve surface of the cap (1).

8. Attachment device for injection needles according to one of the claims 5 to 7, **characterised in that** the cap (1), the spring elements (3) and grooved cams (4) of which are made from a single part, preferably consisting of an injection moulded component.

9. Attachment device for injection needles according to claim 8, **characterised in that** the injection moulding material is a thermoplastic plastic, preferably PCTG (polycyclohexylene-dimethylene-therephtalate).

10. Attachment device for injection needles according to claims 1 to 9, **characterised in that** one end of the needle (2) projects into the cap (1), so that this enters the storage compartment filled with the medication when the cap (1) is pushed onto the thread (5) of the injection unit.

11. Attachment device for injection needles according to claims 1 to 10, **characterised in that** the cap (1) comprises at least five spring elements (3) with grooved cams (4), distributed evenly across the circumference, and has an internal diameter of at least 9 mm.

12. Attachment device for injection needles according to claims 1 to 11, **characterised in that** the needle (2) is a hollow needle with a gauge of less than 30.

## Revendications

1. Dispositif de fixation pour des aiguilles d'injection, destiné à être placé sur un filetage (5) d'un appareil d'injection, le dispositif de fixation étant formé sous la forme d'un capuchon (1) pourvu d'une extrémité ouverte et d'une extrémité fermée maintenant au centre de manière perpendiculaire une aiguille (2), dont la surface enveloppante ayant un léger effet ressort élastique sur le pourtour présente au moins trois ergots (4) orientés en direction du filetage (5), lesquels ergots viennent en prise avec les pas du filetage (7) lors du placement du capuchon (1) sur le filetage (5), **caractérisé en ce que** les trois ergots (4) ou plus forment avec le filetage (5) au moins six points de contact sur au moins deux niveaux.

2. Dispositif de fixation pour des aiguilles d'injection selon la revendication 1, **caractérisé en ce que** chaque ergot (4) a au moins deux pointes (9) venant en prise de manière perpendiculaire avec le filetage (5).

3. Dispositif de fixation pour des aiguilles d'injection selon la revendication 2, **caractérisé en ce que** chaque ergot (4) présente au moins une encoche (8) disposée de manière parallèle par rapport au pourtour enveloppant, les pointes (9) des ergots (4) à encoches formant les six points de contact ou plus sur au moins deux niveaux.

4. Dispositif de fixation pour des aiguilles d'injection selon la revendication 3, **caractérisé en ce que** la distance entre les pointes (9) de chaque ergot (4) à encoches est au moins identique à la hauteur du pas de filetage (7).

5. Dispositif de fixation pour des aiguilles d'injection selon la revendication 3 ou 4, **caractérisé en ce que** la surface enveloppante du capuchon (1) ne peut pas être déformée élastiquement en hauteur, et **en ce que** des éléments formant ressort (3) y sont fixés, lesquels maintiennent les ergots (4) à encoches.

6. Dispositif de fixation pour des aiguilles d'injection selon la revendication 5, **caractérisé en ce que** les éléments formant ressort (3) sont reliés au capuchon (1) dans la zone de l'extrémité supérieure et de l'extrémité inférieure de ce dernier, les éléments formant ressort (3) constituant ce faisant des barres flexibles, au centre desquelles respectivement un ergot (4) à encoches est fixé, dont les pointes (9) viennent systématiquement en prise de manière perpendiculaire avec les pas de filetage (7).

7. Dispositif de fixation pour des aiguilles d'injection selon la revendication 5 ou 6, **caractérisé en ce que** les éléments formant ressort (3) sont des segments de même matière de la surface enveloppante du capuchon (1), qui sont reliés par liaison de matière au capuchon (1) au niveau de l'extrémité supérieure et de l'extrémité inférieure et qui ont une épaisseur de paroi déformable élastiquement, de manière correspondante plus petite que celle de la surface enveloppante solide du capuchon (1), et qui présentent, au centre, des ergots (4) de même matière, à encoches et solides, dont les pointes (9) viennent en prise de manière perpendiculaire avec les pas de filetage (7).

8. Dispositif de fixation pour des aiguilles d'injection selon l'une quelconque des revendications 5 à 7, **caractérisé en ce que** le capuchon (1), ses éléments formant ressort (3) et ses ergots (4) à encoches sont fabriqués à partir d'une même pièce, de préférence sous la forme d'une pièce coulée par injection.

9. Dispositif de fixation pour des aiguilles d'injection selon la revendication 8, **caractérisé en ce que** le matériau de coulée par injection est une matière synthétique thermoplastique, de préférence du PCTG (polycyclohexylène diméthylène téréphtalate).

10. Dispositif de fixation pour des aiguilles d'injection selon la revendication 1 à 9, **caractérisé en ce qu'**une extrémité de l'aiguille (2) dépasse à l'intérieur du capuchon (1) de sorte que cette dernière pénètre, lors du placement du capuchon (1) sur le filetage (5) de l'unité d'injection, dans le réservoir rempli de médicament.

11. Dispositif de fixation pour des aiguilles d'injection selon la revendication 1 à 10, **caractérisé en ce que** le capuchon (1) présente, de manière répartie régulièrement sur le pourtour, au moins cinq éléments formant ressort (3) pourvus d'ergots (4) à encoches et a un diamètre intérieur d'au moins 9 mm.

12. Dispositif de fixation pour des aiguilles d'injection selon la revendication 1 à 11, **caractérisé en ce que** l'aiguille (2) est une aiguille creuse présentant un calibre inférieur au calibre 30.
